# EUROPEAN PATENT SPECIFICATION

(11) **EP 2 188 371 B1**
(45) Date of publication and mention of the grant of the patent: **20.12.2017**
(21) Application number: 08797478.8
(22) Date of filing: 08.08.2008
(51) Int. Cl.: C12P 21/00, C12M 1/00, C12N 5/00

(54) **USE OF PERFUSION TO ENHANCE PRODUCTION OF FED-BATCH CELL CULTURE IN BIOREACTORS**
VERWENDUNG VON PERFUSION ZUR ERHÖHUNG DER PRODUKTION VON FED-BATCH-ZELLKULTUREN IN BIOREAKTOREN
UTILISATION DE LA PERFUSION POUR AMÉLIORER LA PRODUCTION D'UNE CULTURE CELLULAIRE EN MODE SEMI-CONTINU DANS DES BIORÉACTEURS

(30) Priority: 09.08.2007 US 954922 P
(43) Date of publication of application: 26.05.2010
(73) Proprietor: Wyeth LLC, New York, NY 10017-5755 (US)
(72) Inventor: HILLER, Gregory, W., Wakefield, MA 01880 (US)
(74) Representative: Pfizer
(86) International application number: PCT/US2008/072612
(87) International publication number: WO 2009/023562

(56) References cited:
- US-A1- 2006 121 611
- SUN X ET AL: "High-density transient gene expression in suspension-adapted 293 EBNA1 cells" BIOTECHNOLOGY AND BIOENGINEERING 20080101 US, vol. 99, no. 1, 13 July 2007 (2007-07-13), pages 108-116, XP002500586 ISSN: 0006-3592 1097-0290
- JARDIN BARBARA ANN ET AL: "High cell density fed batch and perfusion processes for stable non-viral expression of secreted alkaline phosphatase (SEAP) using insect cells: Comparison to a batch Sf-9-BEV system" BIOTECHNOLOGY AND BIOENGINEERING, vol. 97, no. 2, June 2007 (2007-06), pages 332-345, XP002500587 ISSN: 0006-3592
- KONSTANTINOV KONSTANTIN ET AL: "The "push-to-low" approach for optimization of high-density perfusion cultures of animal cells" ADVANCES IN BIOCHEMICAL ENGINEERING BIOTECHNOLOGY SPRINGER, 233 SPRING STREET, NEW YORK, NY 10013, UNITED STATES SERIES : ADVANCES IN BIOCHEMICAL ENGINEERING / BIOTECHNOLOGY (ISSN 0724-6145(PRINT)), 2006, pages 75-98, XP009107388 ISSN: 3-540-34006-8(H)
- MEUWLY ET AL: "Conversion of a CHO cell culture process from perfusion to fed-batch technology without altering product quality" JOURNAL OF BIOTECHNOLOGY, ELSEVIER SCIENCE PUBLISHERS, AMSTERDAM, NL, vol. 123, no. 1, 3 May 2006 (2006-05-03), pages 106-116, XP005398962 ISSN: 0168-1656
- D. Müller et al.: "Continuous Perfusion versus Discontinuous Fed-Batch" In: E. Lindner-Olsson et al.: "Animal Cell Technology: From Target to Market", 5 July 2006 (2006-07-05), Kluwer Academic Publisher pages 293-299,
- SUNG KWAN YOON ET AL: "Effect of culture temperature on follicle-stimulating hormone production by Chinese hamster ovary cells in a perfusion bioreactor", APPLIED MICROBIOLOGY AND BIOTECHNOLOGY, SPRINGER, BERLIN, DE, vol. 76, no. 1, 3 May 2007 (2007-05-03), pages 83-89, XP019538802, ISSN: 1432-0614, DOI: 10.1007/S00253-007-0985-X
- SIMONE M SCHATZ ET AL: "Higher Expression of Fab Antibody Fragments in a CHU Cell Line at Reduced Temperature", BIOTECHNOLOGY AND BIOENGINEERING, WILEY & SONS, HOBOKEN, NJ, US, vol. 84, no. 4, 20 November 2003 (2003-11-20), pages 433-438, XP007912785, ISSN: 0006-3592, DOI: 10.1002/BIT.10793 [retrieved on 2003-09-11]
- KAZUAKI FURUKAWA ET AL: "Enhancement of productivity of recombinant [alpha]-amidating enzyme by low temperature culture", CYTOTECHNOLOGY, KLUWER ACADEMIC PUBLISHERS, DO, vol. 31, no. 1-2, 1 September 1999 (1999-09-01), pages 85-94, XP019236635, ISSN: 1573-0778, DOI: 10.1023/A:1008059803038
- CHEN Z-L ET AL: "Temperature shift as a process optimization step for the production of pro-urokinase by a recombinant Chinese hamster ovary cell line in high-density perfusion culture", JOURNAL OF BIOSCIENCE AND BIOENGINEERING, ELSEVIER, AMSTERDAM, NL, vol. 97, no. 4, 1 January 2004 (2004-01-01), pages 239-243, XP008105293, ISSN: 1389-1723, DOI: 10.1016/S1389-1723(04)70198-X

## Description

### CROSS-REFERENCE TO RELATED APPLICATIONS

This application claims the benefit of U.S. Provisional Application No. 60/954,922, filed August 9, 2007.

### BACKGROUND OF THE INVENTION

### Field of the Invention

The present invention relates to a method of improving protein production by cultured cells, e.g., animal cells. More specifically, the invention relates to a cell culture method wherein the cells are perfused for a period of time, either continuously or intermittently, and subsequently grown in a fed-batch culture. The method of the invention allows the cell culture to achieve a higher cell density before a protein production phase is initiated. As a result, the quantity of protein produced during the production phase is increased, facilitating, for example, commercial-scale production of the protein. The invention also relates to a perfusion bioreactor apparatus comprising a fresh medium reservoir connected to a bioreactor by a feed pump, a recirculation loop connected to the bioreactor, wherein the recirculation loop comprises a filtration device, e.g., ultrafiltration or microfiltration, and a permeate pump connecting the filtration device to a permeate collection container.

### Related Background Art

A large proportion of biotechnology products, whether commercially available or in development, are protein therapeutics. The cellular machinery of a cell (e.g., an animal cell, a bacterial cell) generally is required to produce many forms of protein therapeutics (e.g., glycosylated proteins, hybridoma-produced monoclonal antibodies). Consequently, there is a large and increasing demand for production of proteins in cell cultures, e.g., in animal cell cultures, and for improved methods related to such production.

As compared to bacterial cell cultures, animal cell cultures have lower production rates and typically generate lower production yields. Thus, a significant quantity of research focuses on animal cell culture conditions and methods that can optimize the polypeptide output, i.e., conditions and methods that support high cell density and high titer of protein. For example, it has been determined that restricted feeding of glucose to animal cell cultures in fed-batch processes controls lactate production without requiring constant-rate feeding of glucose (see U.S. Patent Application Publication No. 2005/0070013).

Two cell culture processes are primarily used for large-scale protein production: the fed-batch process and the perfusion process. The primary goals of these methods is the adding of nutrients, e.g., glucose, as they are being consumed, and the removal of metabolic waste products, e.g., lactic acid and ammonia, as they are being produced. In the fed-batch process, cells receive inoculation medium containing glucose at the initiation of the culture and at one or more points after initiation, but before termination, of the culture. For instance, one fed-batch method is an invariant, constant-rate feeding of glucose (Ljunggren and Häggström (1994) Biotechnol. Bioeng. 44:808-18; Häggström et al. (1996) Annals N.Y. Acad. Sci. 782:40-52). Although this invariant, constant-rate feeding of glucose in a fed-batch process can help control lactic acid production by cultured cells to relatively low levels, maximum cell concentrations, growth rates, and cell viability levels are not achieved (because this method of providing glucose typically results in glucose starvation as cell concentrations increase). Consequently, the quantity of product produced is not optimal.

In the perfusion process, cells also receive inoculation base medium, and at the point when cells achieve a desired cell density, cell perfusion is initiated such that the spent medium is replaced by fresh medium. The perfusion process allows the culture to achieve high cell density, and thus enables the production of a large quantity of product. However, at least some forms of the perfusion process require supplying a large quantity of medium and result in some portion of the product being contained in a large volume of spent medium rather than being concentrated in a single harvest.

Thus, there exists a need for alternative methods of large-scale protein production that maximize cell viability, cell concentration, and the quantity of protein produced, as well as minimize the volume of medium in which the protein product is contained.

### SUMMARY OF THE INVENTION

The present invention provides various methods related to improving protein production in cell cultures, e.g., animal cell cultures, wherein the cell culture is perfused for a period of time, either continuously or intermittently, and subsequently grown in a fed-batch culture. Thus in at least one embodiment, the disclosure provides a method for production of a polypeptide comprising the steps of growing cells in a cell culture to a first critical level; perfusing the cell culture, wherein perfusing comprises replacing spent medium with fresh medium, whereby at least some portion of the cells are retained and at least one waste product is removed; growing cells in the cell culture to a second critical level; initiating a polypeptide production phase; and maintaining cells in a fed-batch culture during at least some portion of the polypeptide production phase. In at least some embodiments of the disclosure, the cell culture is an animal cell culture, e.g., a mammalian cell culture, e.g., a CHO cell culture. The invention provides a cell culture method for production of a polypeptide comprising the steps of:
(a) growing cells in a cell culture to a first critical level wherein said first critical level is reached at
   - a cell density of about 1 million to about 9 millions cells per milliliter,
   - a lactate concentration level of about 1 g/L to about 6g/L or,
   - at day 1 to about day 5 of the cell culture ;
(b) perfusing the cell culture, wherein perfusing comprises replacing spent medium with fresh medium, whereby at least some portion of the cells are retained and at least one waste product is removed;
(c) growing cells in the cell culture to a second critical level wherein said second critical level is reached at
   - a cell density of about 5 million to about 40 million cells per milliliter, or,
   - at about day 2 to about day 7 of the cell culture;
(d) initiating a polypeptide production phase by a change in temperature, pH or osmolality of the cell culture or combination thereof; and
(e) maintaining cells in a fed-batch culture during at least some portion of the polypeptide production phase,
wherein the mammalian cell culture is a CHO cell culture.

In at least some embodiments, the invention provides a method for production of a polypeptide wherein the first critical level is reached at a cell density of about 1 million to about 9 million cells per milliliter, e.g., about 2 million cells per milliliter. In at least some embodiments, the first critical level is reached at a lactate concentration of about 1 g/L to about 6 g/L, e.g., about 2 g/L. In at least some embodiments, the first critical level is reached at about day 1 to about day 5 of the cell culture, e.g., about day 2 of the cell culture. In at least some further embodiments, the first critical level is reached at a cell density of about 1 million to about 9 million cells per milliliter and at a lactate concentration of about 1 g/L to about 6 g/L. In at least some other embodiments, the first critical level is reached at a cell density of about 1 million to about 9 million cells per milliliter and at about day 1 to about day 5 of the cell culture.

In at least some embodiments, the invention provides a method for production of a polypeptide wherein the second critical level is reached at a cell density of about 5 million to about 40 million cells per milliliter, e.g., about 10 million cells per milliliter. In at least some embodiments, the second critical level is reached at about day 2 to about day 7 of the cell culture, e.g., about day 5 of the cell culture. In at least some further embodiments, the second critical level is reached at a cell density of about 5 million to about 40 million cells per milliliter, and at about day 2 to about day 7 of the cell culture.

In at least some embodiments, the invention provides a method for production of a polypeptide wherein the at least one waste product is lactic acid or ammonia. In at least some embodiments, the cell culture is a large-scale cell culture.

In at least some embodiments, the step of initiating the polypeptide production phase comprises a temperature shift in the cell culture. In at least some embodiments, the temperature of the cell culture is lowered from about 37°C to about 31°C.

In at least some embodiments, the invention provides a method for production of a polypeptide wherein the at least one waste product is removed by passing the spent medium through a microfiltration device. In at least some embodiments, the invention further comprises the steps of collecting and purifying the polypeptide from the spent medium. In at least some embodiments, the at least one waste product is removed by passing the spent medium through an ultrafiltration device.

In at least some embodiments, the invention provides a method for production of a polypeptide wherein the step of perfusing comprises continuous perfusion. In at least some embodiments, the step of perfusing comprises intermittent perfusion. In at least some embodiments, the rate of perfusion is constant, or the rate of perfusion is increased or decreased at a steady rate, or the rate of perfusion is increased or decreased in a stepwise manner.

In at least some embodiments, the invention provides a method for production of a polypeptide wherein the step of perfusing is terminated when the cell culture reaches the second critical level. In at least some embodiments, the step of perfusing is continued for a period of time after the cell culture reaches the second critical level, e.g., wherein the period of time is about 2 days.

In at least some embodiments, the invention provides a method for production of a polypeptide wherein the step of perfusing further comprises delivering at least one bolus feed to the cell culture. In at least some embodiments, the invention provides a method for production of a polypeptide wherein the step of maintaining cells in a fed-batch culture is initiated when the cell culture reaches the second critical level. In at least some embodiments, the step of maintaining cells in a fed-batch culture is initiated after a period of time has elapsed since the cell culture reached the second critical level, e.g., wherein the period of time is about 2 days.

In at least some embodiments, the invention provides a method for production of a polypeptide further comprising, after the step of maintaining cells in a fed-batch culture, a step of collecting the polypeptide produced by the cell culture. In at least some embodiments, the invention further comprises, after the step of collecting the polypeptide, a step of purifying the polypeptide. In at least some embodiments, the polypeptide produced by the cell culture is an antibody. In at least some embodiments, the invention provides a method for production of a polypeptide wherein at least one step occurs in a bioreactor.

### BRIEF DESCRIPTION OF THE DRAWINGS

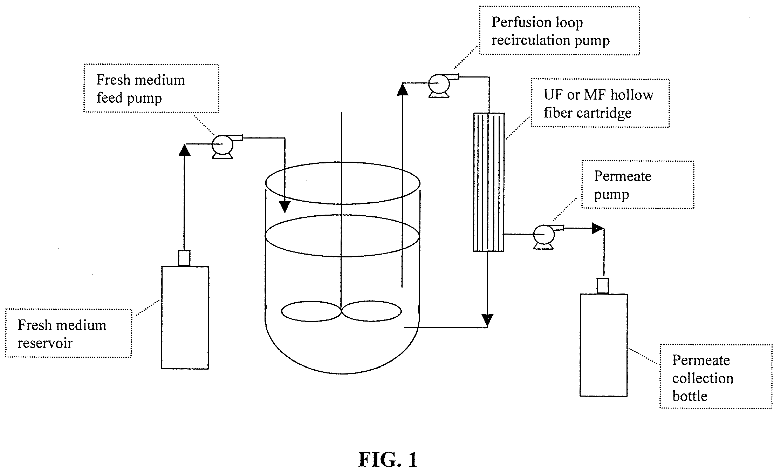

**FIG. 1** demonstrates an exemplary perfusion bioreactor apparatus of the invention, with the ultrafiltration (UF) or microfiltration (MF) device (containing, e.g., a UF or MF hollow fiber cartridge) connected within the external recirculation loop (driven by a perfusion loop recirculation pump).

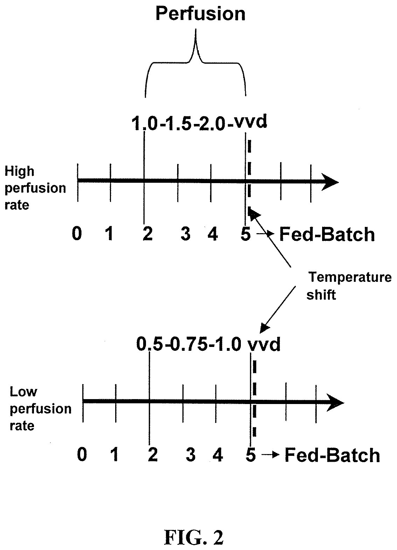

**FIG. 2** represents a time course (in days) for the stepwise increase in perfusion rate for Example 2.2. The upper diagram represents the time course for a 'high perfusion rate' experiment; the lower diagram represents the time course for a 'low perfusion rate' experiment. The perfusion rate was measured in volume per day (vvd); 1.0-2.0 vvd, range for high perfusion rate; 0.5-1.0 vvd, range for low perfusion rate. Numerals (0-5) represent days of culture. Perfusion and fed-batch days were as indicated; dashed lines indicate timing of temperature shift.

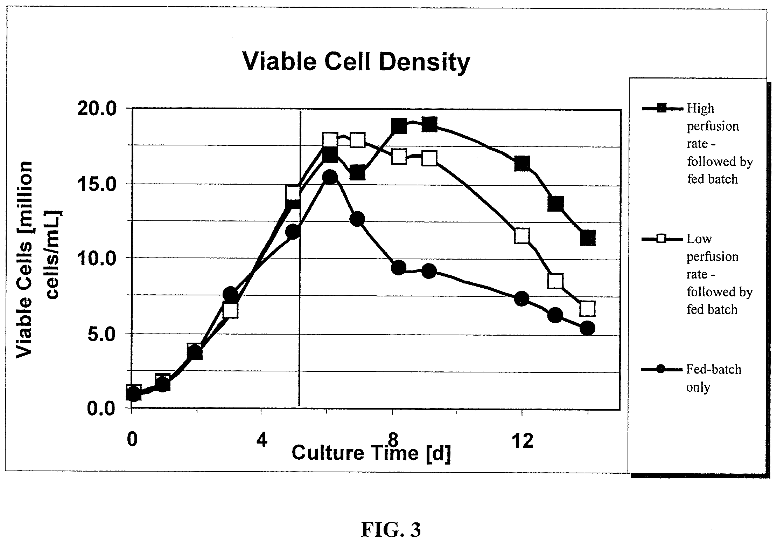

**FIG. 3** demonstrates viable cell density (Y-axis; million cells/mL) at different culture times (X-axis; days [d]) for a high perfusion rate followed by fed-batch culture (■), low perfusion rate followed by fed-batch culture (□), and fed-batch culture only (●) for experiments in Example 2.2. The vertical line on the graph denotes the time at which the temperature was shifted from 37°C to 31°C.

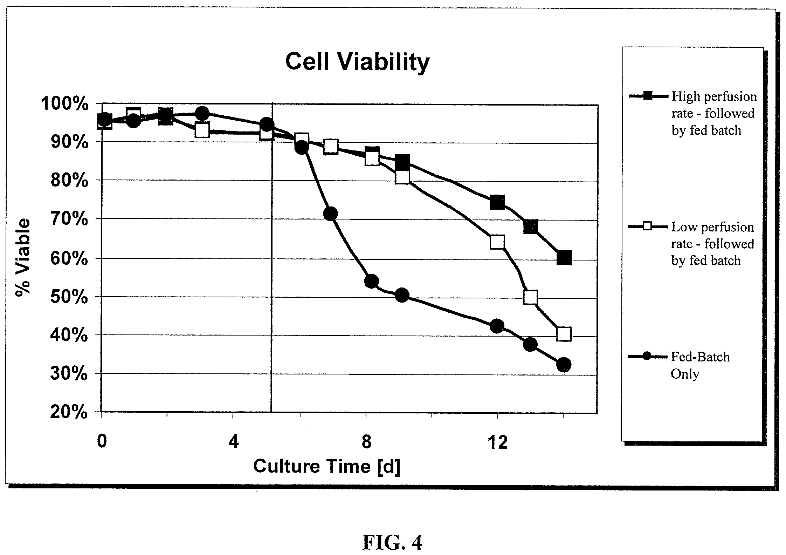

**FIG. 4** demonstrates percent of viable cells (Y-axis) at different culture times (X-axis; days [d]) for a high perfusion rate followed by fed-batch culture (■), a low perfusion rate followed by fed-batch culture (□), and fed-batch culture only (●) for experiments in Example 2.2. The vertical line on the graph denotes the time at which the temperature was shifted from 37°C to 31°C.

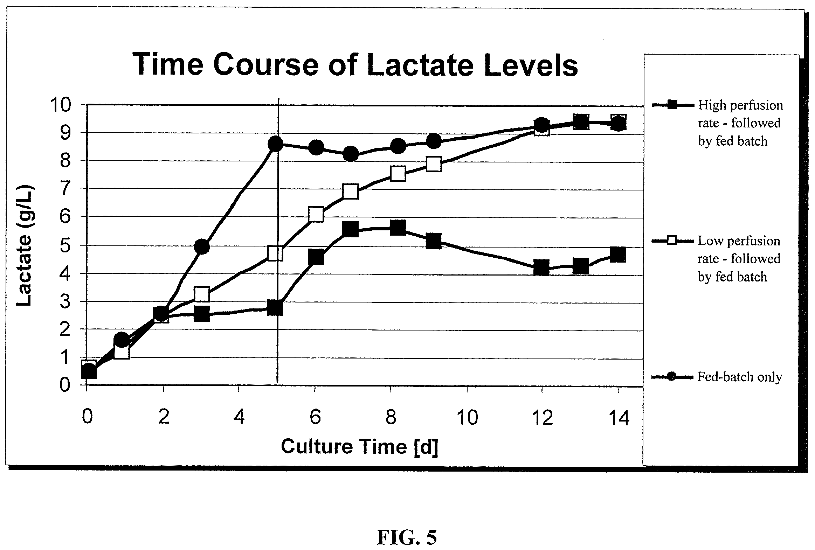

**FIG. 5** demonstrates the concentration of lactate (Y-axis; g/L) for a high perfusion rate followed by fed-batch culture (■), a low perfusion rate followed by fed-batch culture (□), and fed-batch culture only (●) at different culture times (X-axis; days [d]) for experiments in Example 2.2. The vertical line on the graph denotes the time at which the temperature was shifted from 37°C to 31°C.

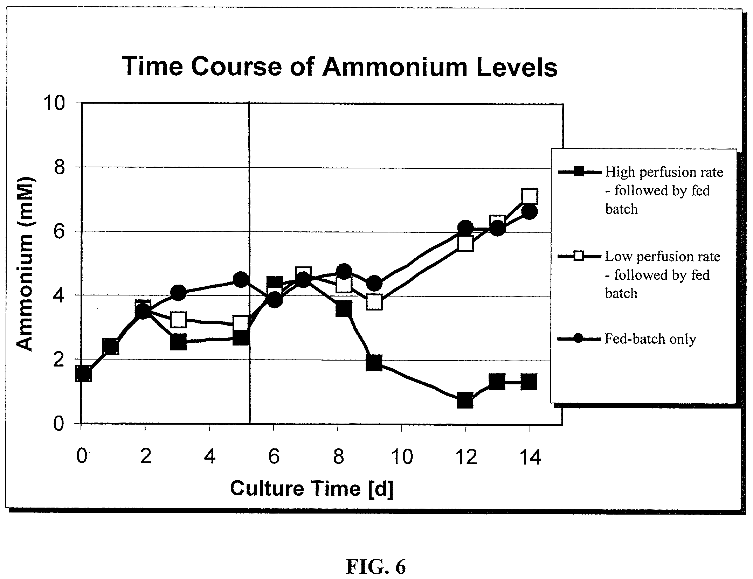

**FIG. 6** demonstrates the concentration of ammonium (Y-axis; mM) for a high perfusion rate followed by fed-batch culture (■), a low perfusion rate followed by fed-batch culture (□), and fed-batch culture only (●) at different culture times (X-axis; days [d]) for experiments in Example 2.2. The vertical line on the graph denotes the time at which the temperature was shifted from 37°C to 31°C.

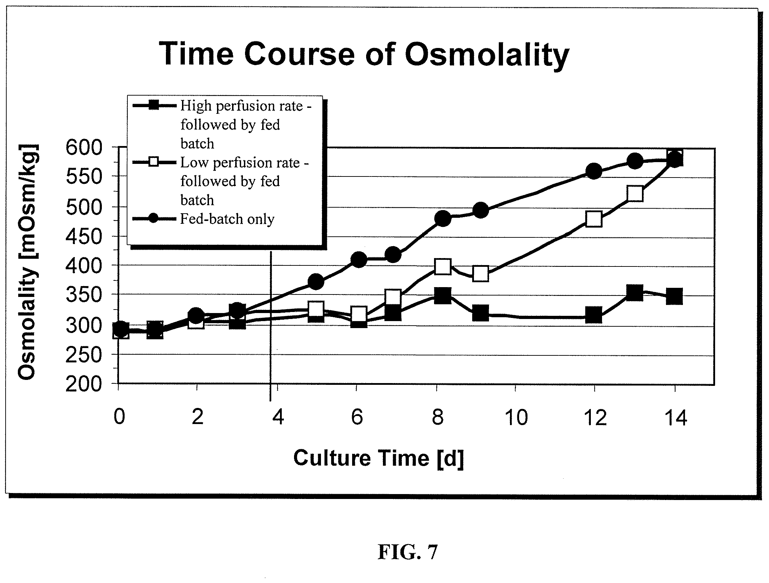

**FIG. 7** demonstrates changes in osmolality (Y-axis; mOsm/kg) for a high perfusion rate followed by fed-batch culture (■), a low perfusion rate followed by fed-batch culture (□), and fed-batch culture only (●) at different culture times (X-axis, days [d]) for experiments in Example 2.2. The vertical line on the graph denotes the time at which the temperature was shifted from 37°C to 31°C.

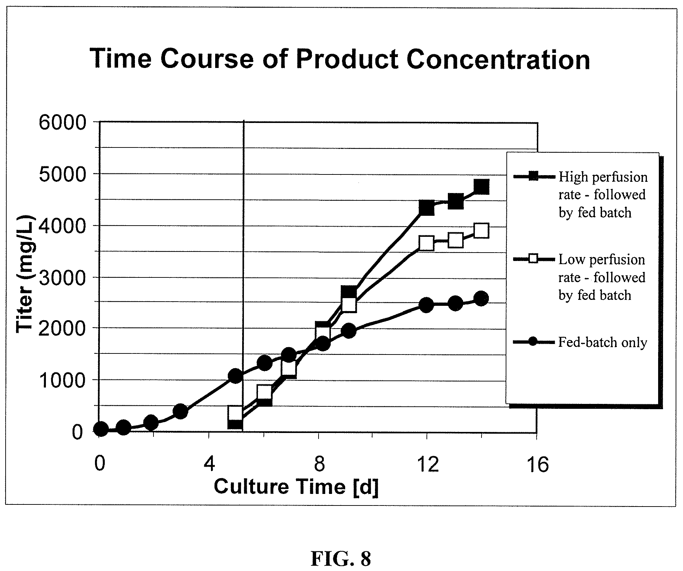

**FIG. 8** demonstrates the titer of monoclonal antibody (Y-axis; mg/L) for a high perfusion rate followed by fed-batch culture (■), a low perfusion rate followed by fed-batch culture (□), and fed-batch culture only (●) at different culture times (X-axis; days [d]) for experiments in Example 2.2. The vertical line on the graph denotes the time at which the temperature was shifted from 37°C to 31°C.

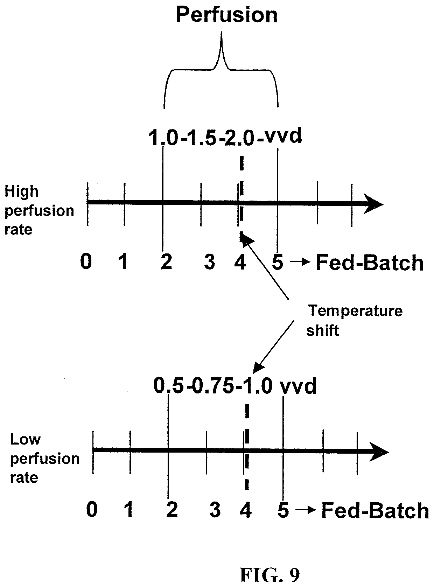

**FIG. 9** represents a time course (in days) for the stepwise increase in perfusion rate for Example 2.3. The upper diagram represents the time course for a high perfusion rate experiment; the lower diagram represents the time course for a low perfusion rate experiment. The perfusion rate was measured in volume per day (vvd); 1.0-2.0 vvd, range for high perfusion rate; 0.5-1.0 vvd, range for low perfusion rate. Numerals (0-5) represent days of culture. Perfusion and fed-batch days were as indicated; dashed lines indicate timing of temperature shift.

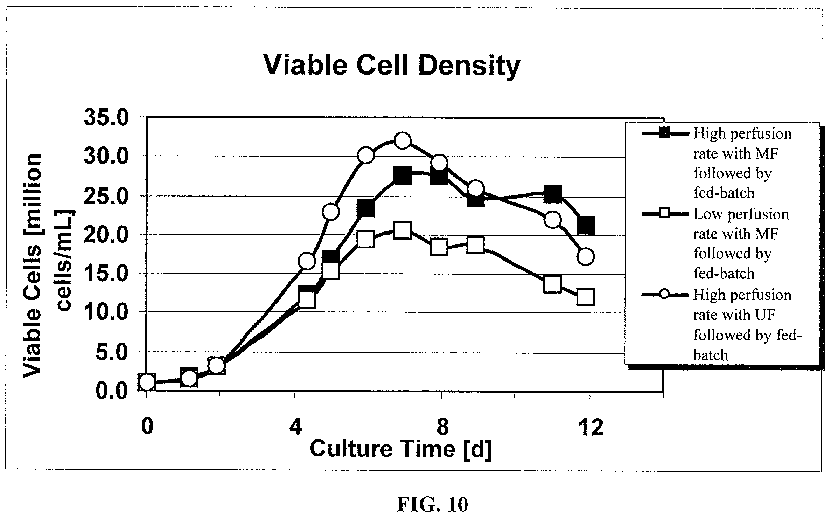

**FIG. 10** demonstrates viable cell density (Y-axis; million cells/mL) at different culture times (X-axis; days [d]) for a high perfusion rate with MF followed by fed-batch culture (■), a low perfusion rate with MF followed by fed-batch culture (□), and a high perfusion rate with UF followed by fed-batch culture (○) for the experiments in Example 2.3. The temperature was shifted from 37°C to 31 °C at approximately day 4.

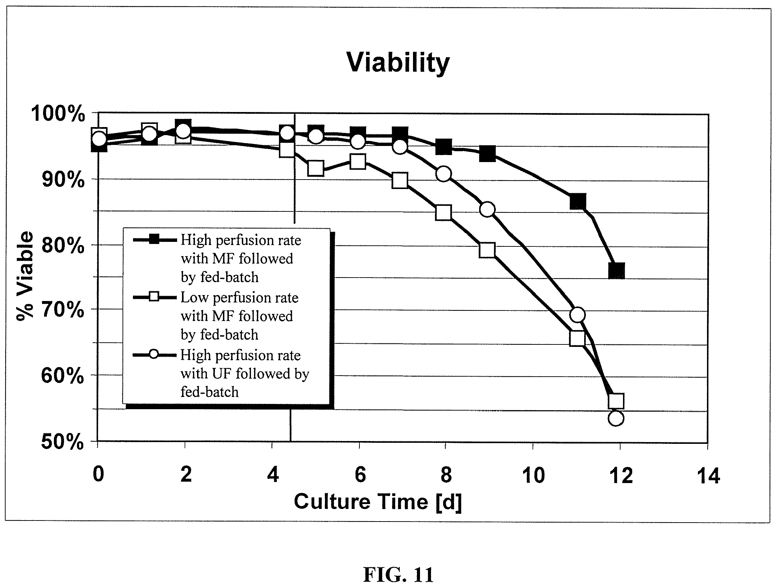

**FIG. 11** demonstrates percent of viable cells (Y-axis) at different culture times (X-axis; days [d]) for a high perfusion rate with MF followed by fed-batch culture (■), a low perfusion rate with MF followed by fed-batch culture (□), and a high perfusion rate with UF followed by fed-batch culture (○) for the experiments in Example 2.3. The vertical line on the graph denotes the time at which the temperature was shifted from 37°C to 31°C.

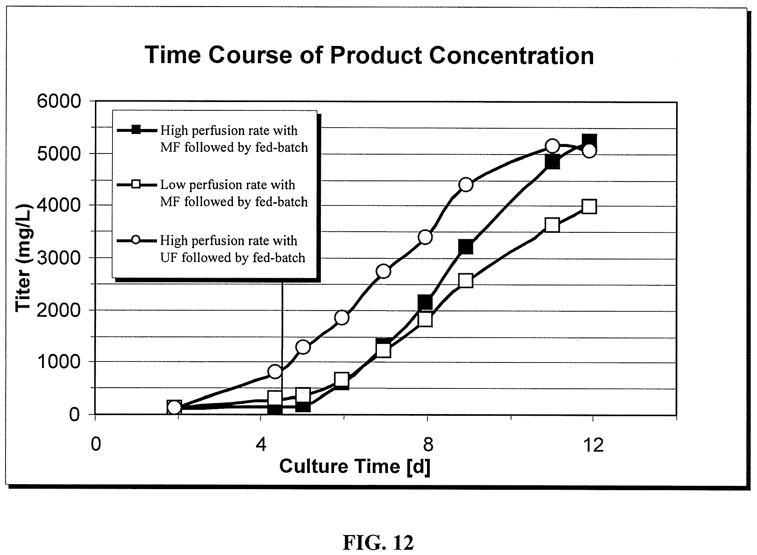

**FIG. 12** demonstrates the titer of monoclonal antibody (Y-axis; mg/L) at different culture times (X-axis; days [d]) for a high perfusion rate with MF followed by fed-batch culture (■), a low perfusion rate with MF followed by fed-batch culture (□), and a high perfusion rate with UF followed by fed-batch culture (○) for the experiments in Example 2.3. The vertical line on the graph denotes the time at which the temperature was shifted from 37°C to 31°C.

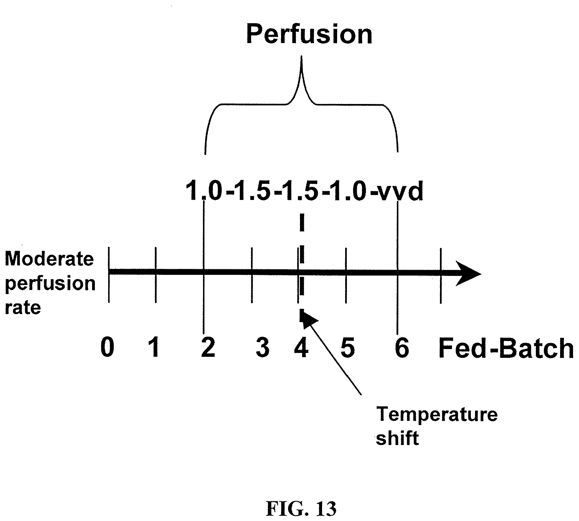

**FIG. 13** represents a time course (in days) for the stepwise changes in perfusion rate ('moderate perfusion rate') for the 'continued' perfusion experiments (perfusion was continued for an additional day as compared with previous experiments) of Example 2.4. Perfusion rate was measured in volume per day (vvd). Numerals (0-6) represent days of culture. Perfusion and fed-batch days were as indicated; the dashed line indicates timing of temperature shift.

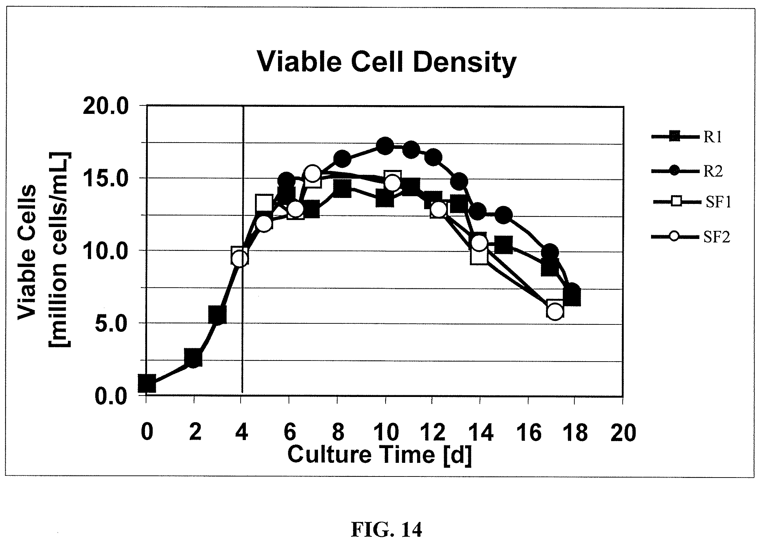

**FIG. 14** demonstrates viable cell density (Y-axis; million cells/mL) at different culture times (X-axis; days [d]) for a moderate perfusion rate culture with MF and normal medium (R1; ■), a moderate perfusion rate culture with UF and concentrated medium (R2; ●); a shake flask containing a sample from R1 (SF1; □); and a shake flask containing a sample from R2 (SF2; ○) for experiments in Example 2.4. The vertical line on the graph denotes the time at which the temperature was shifted from 37°C to 31°C.

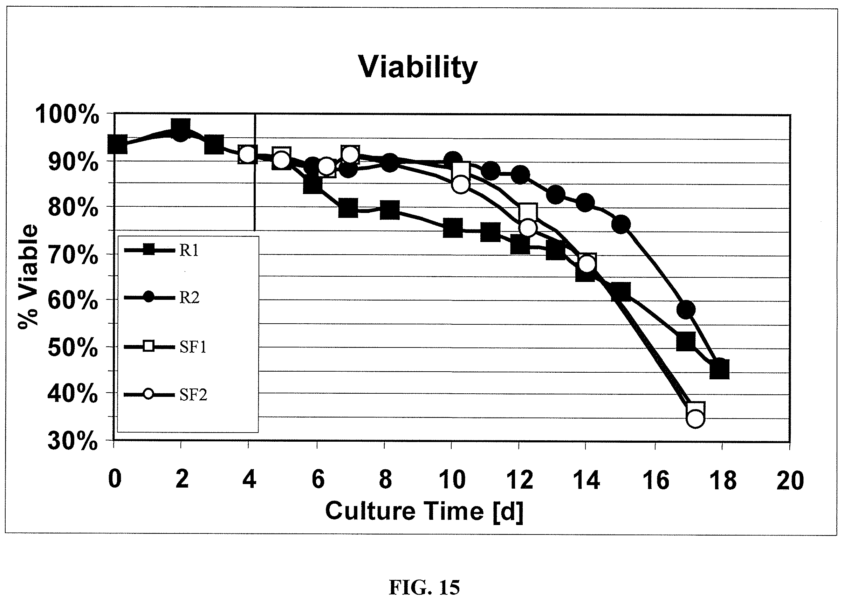

**FIG. 15** demonstrates percent of viable cells (Y-axis) at different culture times (X-axis; days [d]) for a moderate perfusion rate culture with MF and normal medium (R1; ■), a moderate perfusion rate culture with UF and concentrated medium (R2; ●); a shake flask containing a sample from R1 (SF1; □); and a shake flask containing a sample from R2 (SF2; ○) for experiments in Example 2.4. The vertical line on the graph denotes the time at which the temperature was shifted from 37°C to 31°C.

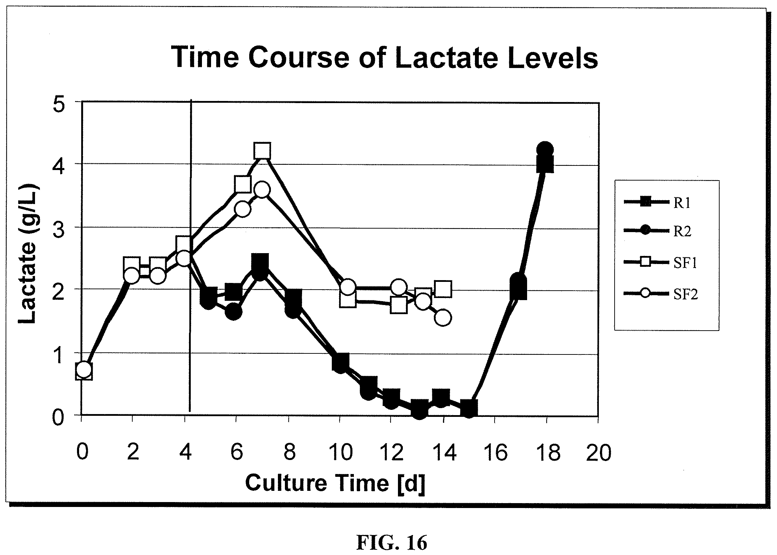

**FIG. 16** demonstrates the concentration of lactate (Y-axis; g/L) at different culture times (X-axis; days [d]) for a moderate perfusion rate culture with MF and normal medium (R1; ■), a moderate perfusion rate culture with UF and concentrated medium (R2; ●); a shake flask containing a sample from R1 (SF1; □); and a shake flask containing a sample from R2 (SF2; ○) for experiments in Example 2.4. The vertical line on the graph denotes the time at which the temperature was shifted from 37°C to 31°C.

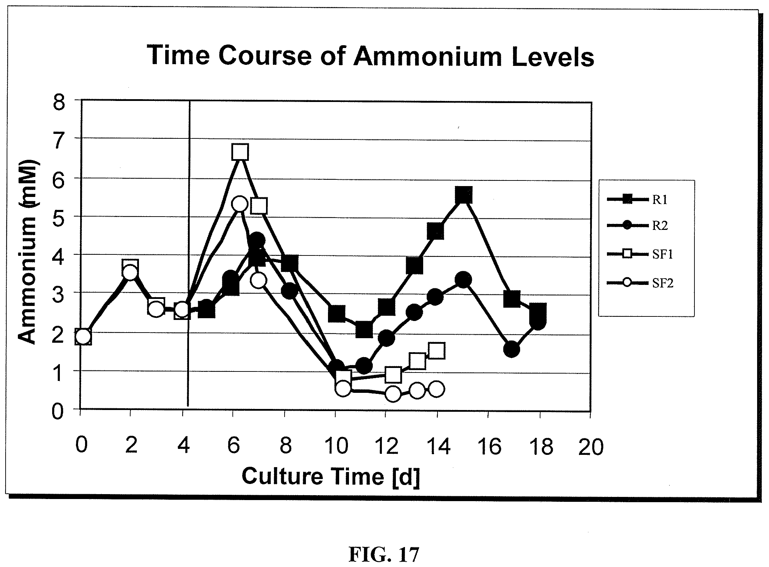

**FIG. 17** demonstrates the concentration of ammonium (Y-axis; mM) at different culture times (X-axis; days [d]) for a moderate perfusion rate culture with MF and normal medium (R1; ■), a moderate perfusion rate culture with UF and concentrated medium (R2; ●); a shake flask containing a sample from R1 (SF1; □); and a shake flask containing a sample from R2 (SF2; ○) for experiments in Example 2.4. The vertical line on the graph denotes the time at which the temperature was shifted from 37°C to 31°C.

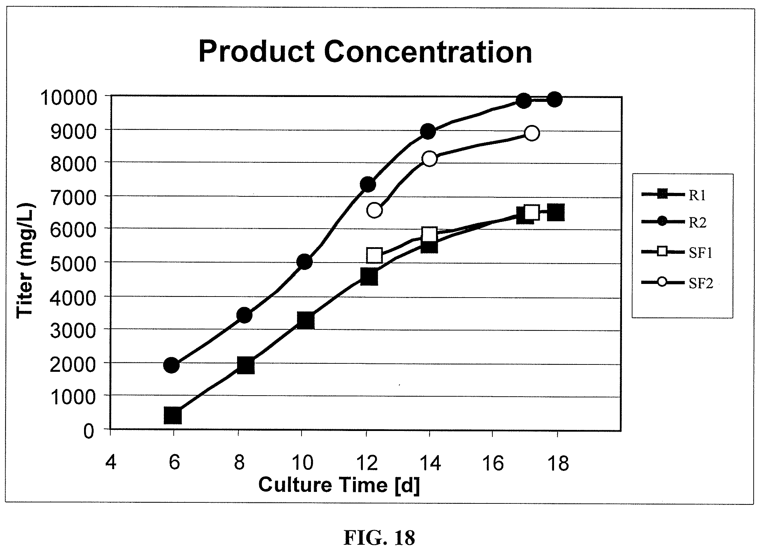

**FIG. 18** demonstrates the titer of monoclonal antibody (Y-axis; mg/L,) for a moderate perfusion rate culture with MF and normal medium (R1; ■), a moderate perfusion rate culture with UF and concentrated medium (R2; ●); a shake flask containing a sample from R1 (SF1; □); and a shake flask containing a sample from R2 (SF2; ○) for experiments in Example 2.4.

### DETAILED DESCRIPTION OF THE INVENTION

The present invention is a modified fed-batch cell culture method for polypeptide production. It provides a method of polypeptide protection, e.g., large-scale polypeptide production, with both increased cell viability and increased quantity of the polypeptide product. The present disclosure also relates to a perfusion bioreactor apparatus that may be used in the disclosed cell culture methods.

The modified fed-batch cell culture method combines both a fed-batch cell culture method and a perfusion method. The terms "culture" and "cell culture" as used herein refer to a cell population that is suspended in a cell culture medium under conditions suitable to survival and/or growth of the cell population. As used herein, these terms may refer to the combination comprising the cell population (e.g., the animal cell culture) and the medium in which the population is suspended.

The term "batch culture" as used herein refers to a method of culturing cells in which all the components that will ultimately be used in culturing the cells, including the medium as well as the cells themselves, are provided at the beginning of the culturing process. A batch culture is typically stopped at some point and the cells and/or components in the medium are harvested and optionally purified.

The term "fed-batch culture" as used herein refers to a method of culturing cells in which additional components are provided to the culture at some time subsequent to the beginning of the culture process. The provided components typically comprise nutritional supplements for the cells that have been depleted during the culturing process. A fed-batch culture is typically stopped at some point and the cells and/or components in the medium are harvested and optionally purified.

The term "perfusion culture" as used herein refers to a method of culturing cells in which additional fresh medium is provided, either continuously over some period of time or intermittently over some period of time, to the culture (subsequent to the beginning of the culture process), and simultaneously spent medium is removed. The fresh medium typically provides nutritional supplements for the cells that have been depleted during the culturing process. Polypeptide product, which may be present in the spent medium, is optionally purified. Perfusion also allows for removal of cellular waste products (flushing) from the cell culture growing in the bioreactor.

The term "bioreactor" as used herein refers to any vessel used for the growth of a prokaryotic or eukaryotic cell culture, e.g., an animal cell culture (e.g., a mammalian cell culture). The bioreactor can be of any size as long as it is useful for the culturing of cells, e.g., mammalian cells. Typically, the bioreactor will be at least 30 ml and may be at least 1, 10, 100, 250, 500, 1000, 2500, 5000, 8000, 10,000, 12,0000 liters or more, or any intermediate volume. The internal conditions of the bioreactor, including but not limited to pH and temperature, are typically controlled during the culturing period. The term "production bioreactor" as used herein refers to the final bioreactor used in the production of the polypeptide or protein of interest. The volume of a large-scale cell culture production bioreactor is generally greater than about 100 ml, typically at least about 10 liters, and may be 500, 1000, 2500, 5000, 8000, 10,000, 12,0000 liters or more, or any intermediate volume. A suitable bioreactor or production bioreactor may be composed of (i.e., constructed of) any material that is suitable for holding cell cultures suspended in media under the culture conditions of the present invention and is conducive to cell growth and viability, including glass, plastic or metal; the material(s) should not interfere with expression or stability of the produced product, e.g., a polypeptide product. One of ordinary skill in the art will be aware of, and will be able to choose, suitable bioreactors for use in practicing the present invention.

The term "cell density" as used herein refers to the number of cells present in a given volume of medium. The term "viable cell density" as used herein refers to the number of live cells present in a given volume of medium under a given set of experimental conditions.

The term "cell viability" as used herein refers to the ability of cells in culture to survive under a given set of culture conditions or experimental variations. The term as used herein also refers to that portion of cells that are alive at a particular time in relation to the total number of cells, living and dead, in the culture at that time.

As used herein, the phrases "polypeptide" or "polypeptide product" are synonymous with the terms "protein" and "protein product," respectively, and, as is generally understood in the art, refer to at least one chain of amino acids linked via sequential peptide bonds. In certain embodiments, a "protein of interest" or a "polypeptide of interest" or the like is a protein encoded by an exogenous nucleic acid molecule that has been transformed into a host cell. In certain embodiments, wherein an exogenous DNA with which the host cell has been transformed codes for the "protein of interest," the nucleic acid sequence of the exogenous DNA determines the sequence of amino acids. In certain embodiments, a "protein of interest" is a protein encoded by a nucleic acid molecule that is endogenous to the host cell. In certain embodiments, expression of such an endogenous protein of interest is altered by transfecting a host cell with an exogenous nucleic acid molecule that may, for example, contain one or more regulatory sequences and/or encode a protein that enhances expression of the protein of interest.

The term "titer" as used herein refers to the total amount of polypeptide of interest produced by a cell culture (e.g., an animal cell culture), divided by a given amount of medium volume; thus "titer" refers to a concentration. Titer is typically expressed in units of milligrams of polypeptide per liter of medium. The modified fed-batch culture of the present invention has an effect of increasing polypeptide product titer compared to other cell culture methods known in the art.

The modified fed-batch cell culture method of the present invention comprises two phases, a cell growth phase and a protein production phase. During the cell growth phase, cells are first mixed (i.e., inoculated) with a medium (i.e., inoculation medium) to form a cell culture. The terms "medium," "cell culture medium," and "culture medium" as used herein refer to a solution containing nutrients that nourish growing animal cells, e.g., mammalian cells, and can also refer to medium in combination with cells. The term "inoculation medium" refers to the medium that is used to form a cell culture. Inoculation medium may or may not differ in composition from the medium used during the rest of the cell growth phase. Typically, medium solutions provide, without limitation, essential and nonessential amino acids, vitamins, energy sources, lipids, and trace elements required by the cell for at least minimal growth and/or survival. The solution may also contain components that enhance growth and/or survival above the minimal rate, including hormones and growth factors. The solution is preferably formulated to a pH and salt concentration optimal for cell survival and proliferation. In at least one embodiment, the medium is a defined medium. Defined media are media in which all components have a known chemical structure. In other embodiments of the invention, the medium may contain an amino acid(s) derived from any source or method known in the art, including, but not limited to, an amino acid(s) derived either from single amino acid addition(s) or from a peptone or protein hydrolysate addition(s) (including animal or plant source(s)). In yet other embodiments of the invention, the medium used during the cell growth phase may contain concentrated medium, i.e., medium that contains higher concentration of nutrients than is normally necessary and normally provided to a growing culture. One skilled in the art will recognize which cell media, inoculation media, etc. is appropriate to culture a particular cell, e.g., animal cell (e.g., CHO cells), and the amount of glucose and other nutrients (e.g., glutamine, iron, trace D elements) or agents designed to control other culture variables (e.g., the amount of foaming, osmolality) that the medium should contain (see, e.g., Mather, J.P., et al. (1999) "Culture media, animal cells, large scale production," Encyclopedia of Bioprocess Technology: Fermentation, Biocatalysis, and Bioseparation, Vol. 2:777-85; U.S. Patent Application Publication No. 2006/0121568). The present invention also contemplates variants of such know media, including, e.g., nutrient-enriched variants of such media.

One skilled in the art will recognize at what temperature and/or concentration a particular cell line should be cultured. For example, most mammalian cells, e.g., CHO cells, grow well within the range of about 35°C to 39°C, preferably at 37°C, whereas insect cells are typically cultured at 27°C.

The present disclosure may use recombinant host cells, e.g., prokaryotic or eukaryotic host cells, i.e., cells transfected with an expression construct containing a nucleic acid that encodes a polypeptide of interest. The phrase "animal cells" encompasses invertebrate, nonmammalian vertebrate (e.g., avian, reptile and amphibian), and mammalian cells. Nonlimiting examples of invertebrate cells include the following insect cells: *Spodoptera frugiperda* (caterpillar), *Aedes aegypti* (mosquito), *Aedes albopictus* (mosquito), *Drosophilia melanogaster* (fruitfly), and *Bombyx mori* (silkworm / silk moth).

A number of mammalian cell lines are suitable host cells for recombinant expression of polypeptides of interest. Mammalian host cell lines include, for example, COS, PER.C6, TM4, VERO076, MDCK, BRL-3A, W138, Hep G2, MMT, MRC 5, FS4, CHO, 293T, A431, 3T3, CV-1, C3H10T1/2, Colo205, 293, HeLa, L cells, BHK, HL-60, FRhL-2, U937, HaK, Jurkat cells, Rat2, BaF3, 32D, FDCP-1, PC12, M1x, murine myelomas (e.g., SP2/0 and NS0) and C2C12 cells, as well as transformed primate cell lines, hybridomas, normal diploid cells, and cell strains derived from *in vitro* culture of primary tissue and primary explants. Any eukaryotic cell that is capable of expressing the polypeptide of interest may be used in the disclosed cell culture methods. Numerous cell lines are available from commercial sources such as the American Type Culture Collection (ATCC). In one embodiment of the disclosure, the cell culture, e.g., the large-scale cell culture, employs hybridoma cells. The construction of antibody-producing hybridoma cells is well known in the art. In one embodiment of the invention, the cell culture, e.g., the large-scale cell culture, employs CHO cells.

Although in certain embodiments the cell culture comprises mammalian cells, one skilled in the art will understand that it is possible to recombinantly produce polypeptides of interest in lower eukaryotes such as yeast, or in prokaryotes such as bacteria. One skilled in the art would know that the culture conditions for yeast and bacterial cell cultures will differ from the culture conditions of animals cells, and will understand how these conditions will need to be adjusted in order to optimize cell growth and/or protein production. One skilled in the art will also know that bacterial or yeast cell culture may produce waste products distinct from mammalian waste products, e.g., ethanol,
acetate, etc.

Suitable yeast strains for polypeptide production include *Saccharomyces cerevisiae, Schizosaccharomyces pombe, Pichia pastoris, Kluyveromyces* strains, *Candida,* or any yeast strain capable of expressing polypeptide of interest. Suitable bacterial strains include *Escherichia coli, Bacillus subtilis, Salmonella typhimurium,* or any bacterial strain capable of expressing the polypeptide of interest. Expression in bacteria may result in formation of inclusion bodies incorporating the recombinant protein. Thus, refolding of the recombinant protein may be required in order to produce active, or more active, material. Several methods for obtaining correctly folded heterologous proteins from bacterial inclusion bodies are known in the art. These methods generally involve solubilizing the protein from the inclusion bodies, then denaturing the protein completely using a chaotropic agent. When cysteine residues are present in the primary amino acid sequence of the protein, it is often necessary to accomplish the refolding in an environment that allows correct formation of disulfide bonds (a redox system). General methods of refolding are known in the art and disclosed in, e.g., Kohno (1990) Meth. Enzymol. 185:187-95, EP 0433225, and U.S. Patent No. 5,399,677.

Subsequent to the forming of the initial cell culture, cells are grown to a first critical level. The term "first critical level" refers to a point during the cell growth phase when the cell viability may be affected by the increased concentration of waste products (e.g., cell growth inhibitors and toxic metabolites, e.g., lactate, ammonium, etc.). In one embodiment on the invention, in an animal cell culture, e.g., a mammalian cell culture, the first critical level is reached at a cell density of about 1 million to about 9 million cells per milliliter, e.g., about 2 million cells per milliliter. In another embodiment of the invention, the first critical level is reached at about day 1 to about day 5 of the cell culture, e.g., at about day 2 of cell culture. In yet another embodiment of the invention, the first critical level is reached at a lactate concentration of about 1 g/L to about 6 g/L, e.g., about 2 g/L. One skilled in the art will be aware that the appropriate levels for such various criteria may differ for other types of cell cultures, e.g., bacterial or yeast cultures.

When the cells reach the first critical level, the perfusion process is initiated. Perfusing a cell culture comprises replacing spent medium (i.e., nutrient-poor, cell free (or nearly cell free), and cell waste product-containing medium) with fresh medium (nutrient-rich medium free of cell waste product(s)), whereby the cells are retained with the use of a cell retention device and the waste products are removed. In one embodiment of the invention, the waste products are removed by passing the medium through a microfiltration (MF) device. In another embodiment of the invention, the waste products are removed by passing the medium through an ultrafiltration (UF) device. Either an MF or a UF device, or the like, is connected to the bioreactor, e.g., within an external recirculation loop that is run parallel to the bioreactor (see, e.g., **FIG. 1** and Example 1). The MF and UF devices can be, e.g., fiber cartridge filters (membranes) that allow certain substances to pass through, while retaining others. In general, MF devices comprise membranes with pore sizes ranging from, e.g., 0.1 to 10 µm; UF devices comprise a range of smaller pore sizes, e.g., the molecular weight cutoff of the membrane for a globular protein may be between 1,000 and 750,000 daltons. Various filtration device setups may be used, e.g., hollow fiber filter plumbed inline, tangential flow filtration device, etc. Such filtration device setups are known to one skilled in the art, as are other forms of cell retention devices that may be used with the present invention, e.g., in the recirculation loop or internal to the bioreactor (see, e.g., Woodside et al. (1998) Cytotechnol. 28:163-75).

As a result of filtration, substances (e.g., waste products, cell debris, etc.) that are small enough to pass through the filter membrane (e.g., MF or UF device), i.e., permeate, can be discarded. Substances that are too large to pass through the filter (e.g., cells, proteins of a certain size, etc.), i.e., retentate, will be retained and, optionally, returned to the bioreactor.

Depending on the method, e.g., a method that allows for both low and high molecular weight substances to pass to the permeate, e.g., the method utilizing a microfiltration device, the permeate may contain product, e.g., a polypeptide product (possibly in low concentration) that can be captured for purification. In such embodiments, the permeate is not discarded but is instead retained and the polypeptide product therefrom is purified, or at least partially purified. Alternatively, the method utilizing the ultrafiltration device simultaneously concentrates and retains the polypeptide product in the bioreactor, so that it can be later collected in a single harvest, possibly simplifying purification of the polypeptide product.

The pore size of the filter determines which substances will pass through to the permeate and which substances will be retained. In one embodiment of the invention, the MF device has 0.2 micron pore size. In another embodiment of the invention, the UF device has a pore size that allows only proteins smaller than 50,000 daltons to pass through to the permeate. Thus, one skilled in the art will recognize that using a UF device with a 50,000 dalton pore size will retain 100% or nearly 100% of the polypeptide product in a large-scale cell culture designed for antibody production (because the molecular weight of an antibody is typically about 150,000 daltons). One skilled in art will also recognize that the pore size of the filter can be varied depending on the size of the final polypeptide product (e.g., in order to retain the optimal amount of the final polypeptide product) or the size of the waste product to be removed.

For example, there may be an additional advantage for maintaining cell viability by retaining cellularly produced proteins other than the polypeptide product, e.g., shear-protective proteins, autocrine growth factors, etc. There also may be a need for removing other proteins, e.g., cell-produced proteases that have accumulated in the culture. Thus, a skilled artisan can adjust the pore size of the filter according to the experimental or production need(s).

In one embodiment of the invention, the fresh medium, which replaces the spent medium during perfusion, is the same medium as inoculation medium. In another embodiment of the invention, the fresh medium may differ from the inoculation medium, e.g., the fresh medium may contain a higher concentration of nutrients.

The rate of perfusion in the present invention can be any rate appropriate to the cell culture. For example, the rate of perfusion can range from about 0.1 vvd to about 20 vvd, or more preferably from about 0.5 vvd to about 10 vvd, or most preferably from about 0.5 vvd to about 2.5 vvd. The rate of perfusion can remain constant over a period of time, or can be altered (i.e., increased or decreased) over the course of a period of perfusion, or any combination thereof. Further, an increase or decrease in the rate of perfusion can be applied in any manner known in the art, including, but not limited to, a steady alteration over time, e.g., a steady increase during a period of perfusion, or a series of alterations over time, e.g., a series of steady alterations, a series of stepwise alterations (e.g., the rate of perfusion could be increased or decreased in a stepwise manner), or any combination thereof. The perfusion can be applied in a continuous manner or in an intermittent manner, as noted above. The timing of the initiation and cessation of a perfusion period(s), and of any alterations to the perfusion, can be predetermined, e.g., at a set time(s) or interval(s), or based upon the monitoring of some parameter or criterion.

The experiments performed herein (Examples) utilized continuous perfusion during the perfusion stage. In continuous perfusion, pumps add fresh medium and remove spent medium continuously from the bioreactor (with no significant change in bioreactor volume), thereby supplying additional nutrients and keeping the concentration(s) of inhibitory substance(s) low. An alternative to continuous perfusion (herein termed "intermittent perfusion") can be useful; for example, if sufficiently high rates of addition / removal of medium can be accomplished, it is possible to perform nearly the same degree of (1) addition of nutrients and (2) removal of inhibitor(s) as accomplished by continuous perfusion in a shorter period of time, e.g., by perfusing the bioreactor for only a fraction of a day (for example, four, six, eight, or ten hours of perfusion per day (i.e., intermittent perfusion) instead of 24 hours per day (continuous)). Such intermittent perfusion can be made possible by, e.g., an oversizing of the filtration / cell retention apparatus in comparison to the size of the bioreactor. Also, alternative technologies including, but not limited to, hydrocyclones (see, e.g., U.S. Patent No. 6,878,545) can be used to make very high rates of perfusion feasible at a large scale (using either continuous or intermittent perfusion as disclosed herein). The ability to perfuse, e.g., several reactor volumes per day in the span of several hours (i.e., intermittent perfusion) can provide several advantages. One advantage is a reduction in the risk of contamination, by virtue of the fact that the perfusion operation would not occur during all shifts of a manufacturing operation. Less shear stress damage to cells due to a reduced number of passages though the cell retention device, and the reduction or elimination of the need for a sterile hold tank for perfusion medium, are two other potential advantages of an intermittent perfusion operation.

Reduction of the volume of a bioreactor prior to an intermittent perfusion is another method for potentially increasing the efficiency of perfusion. For example, before intermittent perfusion, the volume of the bioreactor can be reduced, e.g., by 50% through the removal of spent medium (e.g., cell-free spent medium) without the addition of fresh medium The perfusion can then be performed (with no additional change in bioreactor volume during this phase), and additional medium can later be added to the bioreactor to bring it back to the original volume. If one of skill in the art used the same volume of medium for the entire operation for each of two cases (i.e., perfusion performed with prior reactor volume reduction, compared to perfusion performed without prior reactor volume reduction), and assuming a well-mixed system, basic mathematical calculations dictate that the concentration of any inhibitory compound(s) would be reduced by an additional 50% in the case of the bioreactor with prior volume reduction. Similar calculations can be performed by a skilled artisan to ascertain the value of any particular degree of volume reduction prior to perfusion.

In some embodiments of the invention, it may be necessary to deliver at least one bolus feed to the cell culture during perfusion. The bolus feed is a concentrated nutrient feed, wherein the feed is delivered all at once. In general, such a bolus feed prevents the depletion of nutrients without requiring a modification or adjustment of the composition of the perfusion medium. One skilled in the art would know at what point during cell culture to deliver such a bolus feed(s), e.g., by monitoring nutrient levels in the cell culture.

The step of perfusing the cell culture continues until the cell culture reaches, e.g., a second critical level. The "second critical level" is a point in the growth phase at which the cell density of the cell culture is high, but the practicality of removing cell growth inhibitors and toxic metabolites, e.g., waste products, e.g., lactate and ammonia, by continuing the perfusion becomes limited such that the growth inhibitors and toxic metabolites will begin affecting cell viability and/or productivity. In one embodiment of the invention, in an animal cell culture, e.g., a mammalian cell culture, the second critical level is reached at a cell density of about 5 million to about 40 million cells per milliliter, e.g., about 10 million cells per milliliter. In another embodiment of the invention, the second critical level is reached at about day 2 to about day 7 of cell culture, e.g., about day 5 of cell culture. One skilled in the art will be aware that the appropriate levels for such various criteria may differ for other types of cell cultures, e.g., bacterial or yeast cultures.

At this stage, the perfusion may be either abruptly terminated, or slowly ramped down and continued for some period of time, so that the waste products can continue to be removed. As a result of perfusing the cell culture, toxic components of culture are removed, and the cell growth period is extended, increasing the peak and sustained number of viable cells available for protein production.

When the cell culture reaches the second critical level, the protein production phase is initiated. The production phase is the phase during cell culture, e.g., large-scale cell culture, when the majority of the polypeptide product is produced and collected (although some polypeptide product may have been produced prior to the initiation of the production phase). The production phase is initiated by, for example, a change in, e.g., temperature (i.e., a temperature shift), pH, osmolality, or a chemical or biochemical inductant level of the cell culture, or combinations thereof. The above list is merely exemplary in nature and is not intended to be a limiting recitation. The parameters characteristic of such change, which is sometimes referred to as a metabolic shift, are well known to those skilled in the art. For example, a temperature shift of a CHO cell culture from 37°C to 31°C slows growth of the cell culture and may have an effect of decreasing quantities of lactic acid and ammonia produced by cell culture. Teachings regarding various changes to cell cultures (which may produce a metabolic shift (e.g., a temperature shift)) may be found in the art (see, e.g., U.S. Patent Application Publication No. US 2006/0121568).

A temperature shift can lead to cessation, or near-cessation, of ammonia and lactic acid production. In some cases, late in cell culture, the lactic acid and ammonia may also be consumed by the cell culture. The cessation of the production of lactic acid and ammonia or the consumption of lactic acid and ammonia promote cell viability, cell productivity, and have an effect of increasing polypeptide product titer.

In the present invention, a fed-batch cell culture follows a period(s) of perfusion. Further, the polypeptide production phase follows a metabolic shift, e.g., a temperature shift. As demonstrated in the Examples, the period of perfusion of the cell culture can continue beyond a temperature shift. One skilled in the art would be able to determine the value of continuing the perfusion beyond the temperature shift, or any change to the cell culture that may produce, e.g., a metabolic shift. Thus, a period of fed-batch cell culture may begin at some period of time after, e.g., a temperature shift. At some point during the polypeptide production phase, cells are maintained in a fed-batch cell culture, e.g., once or more than once receiving nutrient feeds. A skilled artisan will recognize that the present invention can be applied to any procedure incorporating fed-batch cell culture, i.e., including the use of any medium appropriate for such cell culture, and including the production of any protein by such cell culture. One skilled in the art also will understand that in some embodiments of the invention, cells maintained in a fed-batch culture may continue to grow and the cell density may continue to increase. In other embodiments, maintaining cells in a fed-batch culture may significantly reduce the rate of the growth of the cells such that the cell density will plateau.

Various fed-batch culture processes are known in the art and can be used in the methods of the present invention. Nonlimiting examples of fed-batch processes to be used with the methods of the present invention include: invariant, constant-rate feeding of glucose in a fed-batch process (Ljunggren and Häggström (1994) Biotechnol. Bioeng. 44:808-18; Häggström et al. (1996) Annals N.Y. Acad. Sci. 782:40-52); a fed-batch process in which glucose delivery is dependent on glucose sampling (e.g., through flow-injection analysis, as by Male et al. (1997) Biotechnol. Bioeng. 55:497-504; Siegwart et al. (1999) Biotechnol. Prog. 15:608-16; or through high-pressure liquid chromatography (HPLC), as by Kurokawa et al. (1994) Biotechnol. Bioeng. 44:95-103); a fed-batch process with rationally designed media (U.S. Patent Application Publication No. 2008/0108553); and a fed-batch process using restricted glucose feed (U.S. Patent Application Publication No. 2005/0070013).

In certain embodiments of the present invention, the practitioner may find it beneficial or necessary to periodically monitor particular conditions of the growing cell culture. Monitoring cell culture conditions allows the practitioner to determine whether the cell culture is producing the recombinant polypeptide of interest at suboptimal levels or whether the culture is about to enter into a suboptimal production phase. Monitoring cell culture conditions also allows the practitioner to determine whether the cell culture requires, e.g., an additional feed. In order to monitor certain cell culture conditions, it may be necessary to remove small aliquots of the culture for analysis. One of ordinary skill in the art will understand that such removal may potentially introduce contamination into the cell culture, and will take appropriate care to minimize the risk of such contamination.

As nonlimiting examples, it may be beneficial or necessary to monitor, e.g., temperature, pH, cell density, cell viability, integrated viable cell density, lactate levels, ammonium levels, osmolality, or titer of the expressed polypeptide. Numerous techniques are well known to those of skill in the art for measuring such conditions/criteria. For example, cell density may be measured using a hemocytometer, an automated cell-counting device (e.g., a Coulter counter, Beckman Coulter Inc., Fullerton, CA), or cell-density examination (e.g., CEDEX®, Innovatis, Malvern, PA). Viable cell density may be determined by staining a culture sample with Trypan blue. Lactate and ammonium levels may be measured, e.g., with the BioProfile 400 Chemistry Analyzer (Nova Biomedical, Waltham, MA), which takes real-time, online measurements of key nutrients, metabolites, and gases in cell culture media. Osmolality of the cell culture may be measured by, e.g., a freezing point osmometer. HPLC can be used to determine, e.g., the levels of lactate, ammonium, or the expressed polypeptide or protein. In one embodiment of the invention, the levels of expressed polypeptide can be determined by using, e.g., protein A HPLC. Alternatively, the level of the expressed polypeptide or protein can be determined by standard techniques such as Coomassie staining of SDS-PAGE gels, Western blotting, Bradford assays, Lowry assays, biuret assays, and UV absorbance. It may also be beneficial or necessary to monitor the post-translational modifications of the expressed polypeptide or protein, including phosphorylation and glycosylation.

At the end of the production phase, the cells are harvested and the polypeptide of interest is collected and purified. Soluble forms of the polypeptide can be purified from conditioned media. Membrane-bound forms of the polypeptide can be purified by preparing a total membrane fraction from the expressing cells and extracting the membranes with a nonionic detergent such as TRITON® X-100 (EMD Biosciences, San Diego, CA). Cytosolic or nuclear proteins may be prepared by lysing the host cells (via mechanical force, Parr-bomb, sonication, detergent, etc.), removing the cell membrane fraction by centrifugation, and retaining the supernatant.

The polypeptide can be purified using other methods known to those skilled in the art. For example, a polypeptide produced by the disclosed methods can be concentrated using a commercially available protein concentration filter, for example, an AMICON® or PELLICON® ultrafiltration unit (Millipore, Billerica, MA). Following the concentration step, the concentrate can be applied to a purification matrix such as a gel filtration medium. Alternatively, an anion exchange resin (e.g., a MonoQ column, Amersham Biosciences, Piscataway, NJ) may be employed; such resin contains a matrix or substrate having pendant diethylaminoethyl (DEAE) or polyethylenimine (PEI) groups. The matrices used for purification can be acrylamide, agarose, dextran, cellulose or other types commonly employed in protein purification. Alternatively, a cation exchange step may be used for purification of proteins. Suitable cation exchangers include various insoluble matrices comprising sulfopropyl or carboxymethyl groups (e.g., S-SEPHAROSE® columns, Sigma-Aldrich, St. Louis, MO).

The purification of the polypeptide from the culture supernatant may also include one or more column steps over affinity resins, such as concanavalin A-agarose, AF-HEPARIN650, heparin-TOYOPEARL® or Cibacron blue 3GA SEPHAROSE® (Tosoh Biosciences, San Francisco, CA); hydrophobic interaction chromatography columns using such resins as phenyl ether, butyl ether, or propyl ether; or immunoaffinity columns using antibodies to the labeled protein. Finally, one or more HPLC steps employing hydrophobic HPLC media, e.g., silica gel having pendant methyl or other aliphatic groups (e.g., Ni-NTA columns), can be employed to further purify the protein. Alternatively, the polypeptides may be recombinantly expressed in a form that facilitates purification. For example, the polypeptides may be expressed as a fusion with proteins such as maltose-binding protein (MBP), glutathione-*S*-transferase (GST), or thioredoxin (TRX); kits for expression and purification of such fusion proteins are commercially available from New England BioLabs (Beverly, MA), Pharmacia (Piscataway, NJ), and Invitrogen (Carlsbad, CA), respectively. The proteins can also be tagged with a small epitope (e.g., His, myc or Flag tags) and subsequently identified or purified using a specific antibody to the chosen epitope. Antibodies to common epitopes are available from numerous commercial sources. Some or all of the foregoing purification steps in various combinations or with other known methods, can be employed to purify a polypeptide of interest produced by the large-scale animal cell culture methods and media described herein.

Methods and compositions of the present invention may be used to produce any protein of interest including, but not limited to, proteins having pharmaceutical, diagnostic, agricultural, and/or any of a variety of other properties that are useful in commercial, experimental and/or other applications. In addition, a protein of interest can be a protein therapeutic. Namely, a protein therapeutic is a protein that has a biological effect on a region in the body on which it acts or on a region of the body on which it remotely acts via intermediates. In certain embodiments, proteins produced using methods and/or compositions of the present invention may be processed and/or modified. For example, a protein to be produced in accordance with the present invention may be glycosylated.

The present invention may be used to culture cells for the advantageous production of any therapeutic protein, such as pharmaceutically or commercially relevant enzymes, receptors, receptor fusions, antibodies (e.g., monoclonal and/or polyclonal antibodies), antigen-binding fragments of an antibody, Fc fusion proteins, cytokines, hormones, regulatory factors, growth factors, coagulation / clotting factors, or antigen-binding agents. The above list of proteins is merely exemplary in nature, and is not intended to be a limiting recitation. One of ordinary skill in the art will know of other proteins that can be produced in accordance with the present invention, and will be able to use methods disclosed herein to produce such proteins.

In one embodiment of the invention, the protein produced using the method of the invention in an antibody or an antigen-binding fragment thereof. As used herein, the term "antibody" includes a protein comprising at least one, and typically two, VH domains or portions thereof, and/or at least one, and typically two, VL domains or portions thereof. In certain embodiments, the antibody is a tetramer of two heavy immunoglobulin chains and two light immunoglobulin chains, wherein the heavy and light immunoglobulin chains are interconnected by, e.g., disulfide bonds. The antibodies, or a portion thereof, can be obtained from any origin, including but not limited to, rodent, primate (e.g., human and nonhuman primate), camelid, shark, etc., or they can be recombinantly produced, e.g., chimeric, humanized, and/or *in vitro*-generated, e.g., by methods well known to those of skill in the art.

Examples of binding fragments encompassed within the term "antigen-binding fragment" of an antibody include, but are not limited to, (i) an Fab fragment, a monovalent fragment consisting of the VL, VH, CL and CH1 domains; (ii) an F(ab')₂ fragment, a bivalent fragment comprising two Fab fragments linked by a disulfide bridge at the hinge region; (iii) an Fd fragment consisting of the VH and CH1 domains; (iv) an Fv fragment consisting of the VL and VH domains of a single arm of an antibody, (v) a dAb fragment, which consists of a VH domain; (vi) a single chain Fv (scFv; see below); (vii) a camelid or camelized heavy chain variable domain (VHH; see below); (viii) a bispecific antibody (see below); and (ix) one or more fragments of an immunoglobulin molecule fused to an Fc region. Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv)); see, e.g., Bird et al. (1988) Science 242:423-26; Huston et al. (1988) Proc. Natl. Acad. Sci. U.S.A. 85:5879-83). Such single chain antibodies are also intended to be encompassed within the term "antigen-binding fragment" of an antibody. These fragments may be obtained using conventional techniques known to those skilled in the art, and the fragments are evaluated for function in the same manner as are intact antibodies.

In some embodiments, the term "antigen-binding fragment" encompasses single domain antibodies. Single domain antibodies can include antibodies whose CDRs are part of a single domain polypeptide. Examples include, but are not limited to, heavy chain antibodies, antibodies naturally devoid of light chains, single domain antibodies derived from conventional four-chain antibodies, engineered antibodies and single domain scaffolds other than those derived from antibodies. Single domain antibodies may be any of those known in the art, or any future single domain antibodies. Single domain antibodies may be derived from any species including, but not limited to, mouse, human, camel, llama, goat, rabbit, bovine, and shark. According to at least one aspect of the invention, a single domain antibody as used herein is a naturally occurring single domain antibody known as heavy chain antibody devoid of light chains. Such single domain antibodies are disclosed in, e.g., International Application Publication No. WO 94/04678. This variable domain derived from a heavy chain antibody naturally devoid of light chain is known herein as a VHH or nanobody, to distinguish it from the conventional VH of four-chain immunoglobulins. Such a VHH molecule can be derived from antibodies raised in *Camelidae* species, for example in camel, Ilama, dromedary, alpaca and guanaco. Other species besides *Camelidae* may produce heavy chain antibodies naturally devoid of light chain; such VHH molecules are within the scope of the invention.

The "antigen-binding fragment" can, optionally, further include a moiety that enhances one or more of, e.g., stability, effector cell function or complement fixation. For example, the antigen-binding fragment can further include a pegylated moiety, albumin, or a heavy and/or a light chain constant region.

Other than "bispecific" or "bifunctional" antibodies, an antibody is understood to have each of its binding sites identical. A "bispecific" or "bifunctional antibody" is an artificial hybrid antibody having two different heavy chain / light chain pairs and two different binding sites. Bispecific antibodies can be produced by a variety of methods including fusion of hybridomas or linking of Fab' fragments; see, e.g., Songsivilai and Lachmann (1990) Clin. Exp. Immunol. 79:315-21; Kostelny et al. (1992) J. Immunol. 148:1547-53. The aforementioned antibodies and antigen-binding fragments may be produced using the methods of the present invention.

In addition, the methods of the present invention can be used to produce small modular immunopharmaceutical (SMIP™) drugs (Trubion Pharmaceuticals, Seattle, WA). SMIPs are single-chain polypeptides composed of a binding domain for a cognate structure such as an antigen, a counterreceptor or the like, a hinge-region polypeptide having either one or no cysteine residues, and immunoglobulin CH2 and CH3 domains (see also www.trubion.com). SMIPs and their uses and applications are disclosed in, e.g., U.S. Patent Application Publication Nos. 2003/0118592, 2003/0133939, 2004/0058445, 2005/0136049, 2005/0175614, 2005/0180970, 2005/0186216, 2005/0202012, 2005/0202023, 2005/0202028, 2005/0202534, and 2005/0238646, and related patent family members thereof.

### EXAMPLES

The Examples which follow are set forth to aid in the understanding of the invention but are not intended to, and should not be construed to, limit the scope of the invention in any way. The Examples do not include detailed descriptions of conventional methods, e.g., cloning, transfection, and basic aspects of methods for overexpressing proteins in cell lines. Such methods are well known to those of ordinary skill in the art.

### Example 1

### Setup of a Perfusion Bioreactor Apparatus

An exemplary bioreactor apparatus of the invention is illustrated in FIG. 1. A stirred-tank bioreactor has an external recirculation loop installed with an MF or UF hollow fiber cartridge filter plumbed inline. The perfusion loop recirculation pump continuously removes cell-containing medium from the bioreactor, pumps it through the tube side of the hollow fiber device, and returns the medium with slightly concentrated cells to the bioreactor. A feed pump delivers fresh medium to the bioreactor and a permeate pump removes cell-free permeate from the shell side of the hollow fiber cartridge filter, maintaining the volume of the bioreactor at an approximately constant level. Depending upon the process, the permeate may contain product that could be captured for purification. The flow rate through the recirculation loop is many times that of the rate at which medium is drawn off by the permeate pump.

### Example 2

### Modified Fed-Batch Process

### Example 2.1: Materials and Methods

A Chinese hamster ovary cell line (CHO-K1), producing a humanized anti-IL-22 monoclonal antibody, was used in the culture experiments. Medium based on at least one formulation included in U.S. Patent Application Publication No. 2006/0121568 was used as perfusion medium in Examples 2.2 and 2.3 ("normal medium" or the like). In Example 2.4, the medium of Examples 2.2 and 2.3 was used for one bioreactor, whereas an additional bioreactor used a nutrient-enriched variant thereof, i.e., medium that was more highly enriched in amino acids and vitamins ("more concentrated medium" or the like). The fed-batch culture portions of the bioreactor experiments also used such media and/or variants thereof. Three-liter (2-liter working volume) Applikon (Foster City, CA) bioreactors with automated controllers (Applikon BioController 1010) were outfitted with external perfusion loops consisting of microfiltration (Spectrum Laboratories, Inc., Rancho Dominguez, CA, 0.2 micron C22M-021-01N) or ultrafiltration (GE Healthcare, Piscataway, NJ, 50 kDa NMWC, model UFP-50-C-5A) hollow fiber cartridges. Culture (containing cells) was circulated to the tube side of the hollow fiber filtration cartridges with a peristaltic pump (Watson-Marlow, Wilmington, MA, model 505U) and cell-free spent medium was removed from the shell side using a ChemTec Tandem model 1081 programmable peristaltic pump (Scilog, Inc., Middleton, WI). Cell density and viability were monitored by the trypan blue dye exclusion method using an automated cell counting device, CEDEX model AS20 (Innovatis GmbH, Bielefeld, Germany). Lactate and ammonium levels were measured using a NOVA Bioprofile 400 automated analyzer (Nova Biomedical Corp., Waltham, MA). Osmolality was measured using an automated osmometer, model 3900 (Advanced Instruments, Inc., Norwood, MA.). Titer (antibody concentration) was measured using Protein A HPLC analytical affinity chromatography (HP Series 1100 HPLC with Applied Biosystems ProA column 2-1001-00, Hewlett-Packard GmbH, Waldbronn, Germany; Applied Biosystems, Foster City, CA).

### Example 2.2: Modified Fed-Batch Process with Microfiltration Device

These experiments investigated the use of continuous perfusion for a relatively short-term followed by fed-batch culture, and used a scheme of stepwise increases in the perfusion rate starting on day 2 of the initial cell culture. The medium used for perfusion was the same medium that was used for the initial inoculation. For experiments labeled 'high perfusion rate' the perfusion of the bioreactor was started at 1 reactor volume per day of perfusion (vvd) on day 2, ramped up to 1.5 vvd the following day, and finally to 2 vvd on day 4, for an additional 24 hours (see **FIG. 2**). At this point, i.e., day 5, the perfusion was stopped, the recirculation through the recirculation loop containing the microfiltration device (hollow fiber 0.2 micron pore size filter) was stopped, and any cells still in the recirculation loop were lost as the recirculation loop was clamped off from the cells in the bioreactor. In other experiments, the 'low perfusion rate' bioreactor started at 0.5 reactor volumes per day of perfusion, and was ramped to 0.75, then 1.0, in a similar manner (**FIG. 2**). A control condition using a fed-batch bioreactor with identical inoculation density and medium was used to determine the extent of any benefit of the continuous, relatively short-term perfusion over a simple fed-batch bioreactor. The temperature in all bioreactors was shifted from 37°C to 31°C on day 5. The fed-batch control culture also received several concentrated feeds of nutrients, starting at day 3, such that the nutrient levels were kept high (to sustain cell growth). Thus, it is likely that the benefits exhibited in the perfusion reactors resulted from the removal of the waste products, e.g., lactate and ammonium.

Significantly higher viable cell densities were reached, and maintained longer, in the bioreactors utilizing the short-term perfusion when compared to the fed-batch control bioreactor (**FIG. 3**). Cell viabilities were also higher, and sustained longer, with short-term perfusion (**FIG. 4**). In addition, a higher perfusion rate extended the viability longer (**FIG. 4**).

Continuous perfusion initiated on day 2 suppressed the accumulation of lactate and ammonium in cell cultures (see **FIGs. 5** and **6**). The high perfusion rate followed by fed-batch culture suppressed lactate and ammonium to a greater extent. The osmotic strength of the medium was also kept in a more suitable range for cell growth and protein production by the introduction of perfusion (**FIG. 7**). The final antibody titer, which correlates with bioreactor productivity, increased about 86% (comparing fed-batch culture only to high perfusion rate culture) through the short-term use of perfusion (**FIG. 8**). The final antibody titer recovered from the perfusion bioreactor was lower than the actual antibody produced in the perfusion bioreactor, as some antibody was lost in the permeate and was not collected or recovered.

### Example 2.3: Modified Fed-Batch Process with Ultrafiltration Device

In these experiments, stepwise increases in the perfusion rate were initiated on day 2 of the cell culture, and the temperature shift from 37°C to 31°C was performed on day 4 (**FIG. 9**). On day 5, perfusion was stopped and cells were maintained as a fed-batch cell culture. No fed-batch control was performed for this experiment. An additional experimental condition consisted of a bioreactor operating at high perfusion rate, except the recirculation loop contained an ultrafiltration device (UF) hollow fiber with a cut-off of 50,000 daltons. This device retained nearly 100% of the polypeptide product (i.e., the anti-IL-22 antibody). Cell densities in these experiments were significantly higher than the cell densities of cultures in Example 2.2 (see **FIG. 10****;** cf. **FIG. 3**). The bioreactor connected to the UF device performed similarly, if not better than, the bioreactor connected to the MF device. It is worth noting that there was no plugging, i.e., reduction in permeate flow, observed in the recirculation loop (i.e., cell-retention device), possibly due to the high cell viabilities achieved in both the bioreactor operating with the MF device and the bioreactor operating with the UF device (**FIG**. **11**). Very high antibody titers were achieved; for example, the bioreactor operating with the UF device reached 4.5 g/L antibody concentration in only nine days (see **FIG**. **12**).

### Example 2.4: Modified Fed-Batch Process with Continued Perfusion

In the "continued" (i.e., extended) perfusion experiment, perfusion was extended to day 6 of the culture, with maximal perfusion flow rate at 1.5 vvd (see **FIG**. **13**). One bioreactor used normal medium and had the recirculation loop attached to a MF device (R1), while another bioreactor used a more concentrated medium formulation and had the recirculation loop attached to a UF device (R2).

To determine the utility of the continued perfusion (e.g., perfusion extending two days beyond the time of temperature shift), samples were removed from the bioreactors on the day of temperature shift, i.e., day 4, and placed in Erlenmeyer-style plastic shake flasks in a humidified incubator with 7% carbon dioxide at 31°C. Shake flask 1 (SF1) contained samples from R1 and shake flask 2 (SF2) contained samples from R2. Such shake flasks are generally known to yield results similar to the controlled conditions of the stirred tank bioreactor. While the flasks were no longer perfused, they were fed with concentrated nutrients in a similar manner to the stirred tank bioreactors.

In addition, bolus nutrient feeds to the bioreactors in this experiment were initiated on day 5, preceding the cessation of perfusion (see **FIG. 13**). The feeds were also more frequent and more concentrated than in Examples 2.2 and 2.3. To approximate the levels of nutrients remaining in cell culture, bioreactor samples were tested regularly by a freezing point osmometer for osmotic strength. If the nutrient feed from the previous day had been largely consumed, i.e., the osmotic strength had returned to prefeeding value, the culture was supplemented with an additional feed.

The cell densities in this experiment were not as high as in Example 2.3, but the viability was sustained for much longer, resulting in higher integrated viable cell density (data not shown). By comparing the viable cell number in the samples in the shake flasks to those of the bioreactors from which they were removed, it was apparent that the continued perfusion, i.e., perfusion for two days beyond the temperature shift on day 4, slightly increased the peak viable cell density in the bioreactor utilizing the more concentrated medium and the UF cell retention device, but did not appear to significantly benefit the viable cell density in the bioreactor utilizing the less concentrated medium and the MF cell retention device (see **FIG. 14**). It is possible that the shear stress from the continued recirculation of cells though the MF filtration loop slightly decreased the viability of the culture maintained in the bioreactor when compared to that of the shake flask from days 4 to 6 (see **FIG. 15**).

The continued perfusion in the bioreactors suppressed the accumulation of lactate and ammonium, compared to the levels in the nonperfused shake flasks, from days 4-6 (see **FIGs. 16** and **17**). However, the cells in the nonperfused shake flasks still converted their metabolism, and began to take up lactic acid and ammonia around day 6 or 7.

The product concentrations for this experiment are shown in **FIG. 18****.** For all conditions, the titers are higher than any that have been reported in the literature to date for a fed-batch mammalian cell culture process. The UF condition with the concentrated medium achieved 8.9 g/l on day 14, and 9.9 g/l on day 17. There was only a slight difference in the concentration of product in the nonperfused shake flasks, suggesting that the perfusion beyond day 4 may not have been necessary to achieve high bioreactor productivity. It is worth noting that, if a UF device were used as the cell retention methodology, there would also be no increase in harvest volume, which is a consideration for a facility with fixed processing tank volumes (e.g., no increase in harvest volume would simplify purification).

## Claims

1. A cell culture method for production of a polypeptide comprising the steps of:
(a) growing cells in a cell culture to a first critical level wherein said first critical level is reached at
- a cell density of 1 million to 9 millions cells per milliliter,
- a lactate concentration level of 1 g/L to 6g/L or,
- at day 1 to day 5 of the cell culture ;
(b) perfusing the cell culture, wherein perfusing comprises replacing spent medium with fresh medium, whereby at least some portion of the cells are retained and at least one waste product is removed;
(c) growing cells in the cell culture to a second critical level wherein said second critical level is reached at
- a cell density of 5 million to 40 million cells per milliliter, or,
- at day 2 to day 7 of the cell culture;
(d) initiating a polypeptide production phase by a change in temperature, pH or osmolality of the cell culture or combination thereof; and
(e) maintaining cells in a fed-batch culture during at least some portion of the polypeptide production phase,
wherein the cell culture is a CHO cell culture.

2. The method of claim 1, wherein the first critical level is reached at a cell density of 1 million to 9 million cells per milliliter.

3. The method of claim 1, wherein the first critical level is reached at a lactate concentration of 1 g/L to 6 g/L.

4. The method of claim 1, wherein the first critical level is reached at day 1 to day 5 of the cell culture.

5. The method of claim 1, wherein the second critical level is reached at a cell density of 5 million to 40 million cells per milliliter.

6. The method of claim 1, wherein the second critical level is reached at day 2 to day 7 of the cell culture.

7. The method of claim 1, wherein the cell culture is a large-scale cell culture.

8. The method of claim 1, wherein the step of initiating the polypeptide production phase comprises a temperature shift in the cell culture.

9. The method of claim 1, wherein the step of perfusing further comprises delivering at least one bolus feed to the cell culture.

10. The method of claim 1, wherein the step of maintaining cells in a fed-batch culture is initiated when the cell culture reaches the second critical level.

11. The method of claim 1, wherein the step of maintaining cells in a fed-batch culture is initiated after a period of time has elapsed since the cell culture reached the second critical level.

12. The method of claim 1, further comprising, after the step of maintaining cells in a fed-batch culture, a step of collecting the polypeptide produced by the cell culture.

13. The method of claim 12, further comprising, after the step of collecting the polypeptide, a step of purifying the polypeptide.

14. The method of any one of claims 1, 7, 12 and 13, wherein the polypeptide produced by the cell culture is an antibody.

## Patentansprüche

1. Zellkulturverfahren zur Herstellung eines Polypeptids, umfassend die Schritte:
(a) Kultivierung der Zellen einer Zellkultur bis zu einem ersten kritischen Level, wobei das erste kritische Level erreicht ist bei
- einer Zelldichte von 1 Million bis 9 Millionen Zellen pro Milliliter
- einem Lactatkonzentrationslevel von 1 g/L bis 6 g/L oder
- an Tag 1 bis Tag 5 der Zellkultur;
(b) Perfundieren der Zellkultur, wobei das Perfundieren umfasst: ein Ersetzen des verbrauchten Mediums mit frischem Medium, wobei mindestens ein gewisser Teil der Zellen zurückgehalten wird und wenigstens ein Abfallprodukt entfernt wird;
(c) Kultivierung der Zellen in einer Zellkultur bis zu einem zweiten kritischen Level, wobei das zweite kritische Level erreicht ist bei
- einer Zelldichte von 5 Millionen bis 40 Millionen Zellen pro Milliliter oder
- an Tag 2 bis Tag 7 der Zellkultur
(d) Initiieren einer Polypeptidproduktionsphase durch eine Änderung der Temperatur, des pH, oder der Osmolalität der Zellkultur oder einer Kombination davon, und
(e) Aufrechterhalten der Zellen in einer Fed-Batch-Kultur während wenigstens eines gewissen Teils der Polypeptidproduktionsphase, wobei die Zellkultur eine CHO-Zellkultur ist.

2. Verfahren gemäß Anspruch 1, wobei das erste kritische Level erreicht ist bei einer Zelldichte von 1 Million bis 9 Millionen Zellen pro Milliliter.

3. Verfahren gemäß Anspruch 1, wobei das erste kritische Level erreicht ist bei einer Lactatkonzentration von 1 g/L bis 6 g/L.

4. Verfahren gemäß Anspruch 1, wobei das erste kritische Level erreicht ist an Tag 1 bis Tag 5 der Zellkultur.

5. Verfahren gemäß Anspruch 1, wobei das zweite kritische Level erreicht ist bei einer Zelldichte von 5 Millionen bis 40 Millionen Zellen pro Milliliter.

6. Verfahren gemäß Anspruch 1, wobei das zweite kritische Level erreicht ist an Tag 2 bis Tag 7 der Zellkultur.

7. Verfahren gemäß Anspruch 1, wobei die Zellkultur eine Zellkultur in großem Maßstab ist.

8. Verfahren gemäß Anspruch 1, wobei der Schritt der Initiierung der Polypeptidproduktionsphase eine Temperaturänderung in der Zellkultur umfasst.

9. Verfahren gemäß Anspruch 1, wobei der Schritt des Perfundierens zusätzlich umfasst: die Abgabe von wenigstens einem Bolus Nährstoffen an die Zellkultur.

10. Verfahren gemäß Anspruch 1, wobei der Schritt des Aufrechterhaltens der Zellen in einer Fed-Batch-Kultur initiiert wird, wenn die Zellkultur das zweite kritische Level erreicht.

11. Verfahren gemäß Anspruch 1, wobei der Schritt des Aufrechterhaltens der Zellen in einer Fed-Batch-Kultur initiiert wird, nachdem ab Erreichen des zweiten kritischen Levels ein Zeitraum vergangen ist.

12. Verfahren gemäß Anspruch 1, zusätzlich umfassend nach dem Schritt des Aufrechterhaltens der Zellen in einer Fed-Batch-Kultur einen Schritt des Sammelns des Polypeptids, welches durch die Zellkultur produziert wird.

13. Verfahren gemäß Anspruch 12, weiterhin umfassend nach dem Schritt des Sammelns des Polypeptids einen Schritt der Reinigung des Polypeptids.

14. Verfahren gemäß einem der Ansprüche 1, 7, 12 und 13, wobei das Polypeptid, welches durch die Zellkultur produziert wird, ein Antikörper ist.

## Revendications

1. Procédé de culture cellulaire pour la production d'un polypeptide comprenant les étapes de :
(a) croissance de cellules dans un milieu de culture cellulaire à un premier niveau critique, dans lequel ledit premier niveau critique est atteint à
- une densité de cellules de 1 million à 9 millions de cellules par millilitre,
- un niveau de concentration en lactate de 1 g/L à 6 g/L ou,
- au jour 1 jusqu'au jour 5 de la culture cellulaire ;
(b) perfusion de la culture cellulaire, dans lequel la perfusion comprend le remplacement du milieu usé avec un milieu frais, moyennant quoi au moins une portion des cellules est retenue et au moins un produit de déchet est enlevé ;
(c) croissance de cellules dans la culture cellulaire à un second niveau critique, dans lequel ledit second niveau critique est atteint à
- une densité de cellules de 5 millions à 40 millions de cellules par millilitre, ou,
- au jour 2 jusqu'au jour 7 de la culture cellulaire ;
(d) initiation d'une phase de production de polypeptides par un changement de température, de pH ou d'osmolalité de la culture cellulaire ou une combinaison de ceux-ci ; et
(e) maintien de cellules dans une culture en cuvée alimentée pendant au moins une partie de la phase de production de polypeptide,
dans lequel la culture cellulaire est une culture cellulaire de CHO.

2. Procédé selon la revendication 1, dans lequel le premier niveau critique est atteint à une densité de cellules de 1 million à 9 millions de cellules par millilitre.

3. Procédé selon la revendication 1, dans lequel le premier niveau critique est atteint à une concentration en lactate de 1 g/L à 6 g/L.

4. Procédé selon la revendication 1, dans lequel le premier niveau critique est atteint au jour 1 jusqu'au jour 5 de la culture cellulaire.

5. Procédé selon la revendication 1, dans lequel le second niveau critique est atteint à une densité de cellules de 5 millions à 40 millions de cellules par millilitre.

6. Procédé selon la revendication 1, dans lequel le second niveau critique est atteint au jour 2 jusqu'au jour 7 de la culture cellulaire.

7. Procédé selon la revendication 1, dans lequel la culture cellulaire est une culture cellulaire à grande échelle.

8. Procédé selon la revendication 1, dans lequel l'étape d'initiation de la phase de production de polypeptide comprend un décalage de température dans la culture cellulaire.

9. Procédé selon la revendication 1, dans lequel l'étape de perfusion comprend en outre l'apport d'au moins une alimentation de bolus à la culture cellulaire.

10. Procédé selon la revendication 1, dans lequel l'étape de maintien de cellules dans une culture en cuvée alimentée est initiée lorsque la culture cellulaire atteint le second niveau critique.

11. Procédé selon la revendication 1, dans lequel l'étape de maintien de cellules dans une culture en cuvée alimentée est initiée après qu'une période de temps s'est écoulée depuis que la culture cellulaire a atteint le second niveau critique.

12. Procédé selon la revendication 1, comprenant en outre après l'étape de maintien de cellules dans une culture en cuvée alimentée, une étape de collecte du polypeptide produit par la culture cellulaire.

13. Procédé selon la revendication 12, comprenant en outre, après l'étape de collecte du polypeptide, une étape de purification du polypeptide.

14. Procédé selon l'une quelconque des revendications 1, 7, 12 et 13 ; dans lequel le polypeptide produit par la culture cellulaire est un anticorps.
